# EUROPÄISCHE PATENTSCHRIFT

(11) **EP 0 796 839 B1**
(45) Veröffentlichungstag und Bekanntmachung des Hinweises auf die Patenterteilung: **09.02.2000**
(21) Anmeldenummer: 97103612.4
(22) Anmeldetag: 05.03.1997
(51) Int. Cl.: C07C 209/72

(54) **Verfahren zur Herstellung eines Gemisches von Amino-methyl-cyclohexanen und Diamino-methyl-cyclohexanen**
Process for the preparation of a mixture of amino-methyl-cyclohexanes and diamino-methyl-cyclohexanes
Procédé pour la préparation d'un mélange d'amino-méthyl-cyclohexanes et de diamino-méthyl-cyclohexanes

(30) Priorität: 18.03.1996 DE 19610545
(43) Veröffentlichungstag der Anmeldung: 24.09.1997
(73) Patentinhaber: BAYER AG, 51368 Leverkusen (DE)
(72) Erfinder: Darsow, Gerhard, Dr., 47804 Krefeld (DE); Petruck, Gerd-Michael, Dr., 40699 Erkrath (DE)

(56) Entgegenhaltungen:
- EP-A- 0 351 661
- EP-A- 0 668 101

## Beschreibung

Die Erfindung betrifft ein kontinuierliches Verfahren zur Herstellung eines Gemisches aus Amino-methyl-cyclohexanen und Diamino-methyl-cyclohexanen in variablen Mengen durch katalytische Hydrierung von Diamino-toluolen mit Wasserstoff bei erhöhter Temperatur unter Einsatz eines Ruthenium-Katalysators auf einem mit Verbindungen von Seltenerdmetallen, von Mangan sowie von Alkalimetallhydroxiden oder von Erdalkalimetallhydroxiden behandelten Al₂O₃-Träger im Festbett.

Amino-methyl-cyclohexane finden Verwendung zur Herstellung von Alterungsschutzmitteln für Kautschuke und Kunststoffe, als Korrosionsschutzmittel sowie als Vorprodukte für Textilhilfsmittel und Pflanzenschutzmittel.

Diamino-methyl-cyclohexane finden Verwendung zur Herstellung von Pulverlackhärtern, Epoxidhärtern, lichtechten Lackharzen sowie wäßrigen Lackharzdispersionen.

Es ist bekannt, Diamino-methyl-cyclohexane durch Druckhydrierung von Diamino-toluolen herzustellen. Für diese Hydrierung in Batch-Fahrweise werden als Katalysatoren Nickel-Legierungen mit Molybdän, Ruthenium, Tantal, Titan (EP 091 028) oder Kobaltoxid (DE 16 18 638/ DE 2 024 858 / JP 71-64 898) eingesetzt.

Andere Verfahren in Batch-Fahrweise verwenden Edelmetalle auf Trägern, wie Rhodium auf Al₂O₃ (JP 83-89 242), Platin und Palladium auf Kohle (US 3 520 928) oder Ruthenium auf Al₂O₃, SiO₂ bzw. Kohle (DE 2 132 547 / JP 74-141 907) oder Ruthenium, Chrom und Mangan auf Al₂O₃ (DE-OS 2 502 893 / DE-OS 2 745 172), wobei in den Versuchsbeispielen ausschließlich diskontinuierliche Verfahren beschrieben sind und als Reaktionsprodukte ausschließlich Diamino-methyl-cyclohexane genannt werden.

Amino-methyl-cyclohexane entstehen bei diesen Reaktionen offenbar überhaupt nicht. Um Amino-methyl-cyclohexane in größeren Mengen zu erhalten, werden sie nach separaten Verfahren hergestellt. So wird z.B. 1-Amino-4-methyl-cyclohexan durch Hydrierung von p-Toluidin an einem Palladium (Platin)/Kohle-Katalysator hergestellt (US 3 520 928).

EP-A 0 668 101 beschreibt Ruthenium-Katalysatoren, die zusätzlich Carbonat, Hydroxide und/oder Oxidhydrate des Cers und Mangans oder des Mangans und/oder deren Dehydratisierungsprodukte enthalten und zur Hydrierung von aromatischen Aminen zu cycloaliphatischen Polyaminen eingesetzt werden.

Ein gemeinsames Problem der Verfahren zur Kernhydrierung methyl-substituierter aromatischer Amine besteht in der z.T. beträchtlichen Bildung von methyl- und aminosubstituierten Dicyclohexyl-aminen als unbrauchbaren Nebenprodukten. Es besteht daher der Wunsch, ein kontinuierliches auch im technischen Maßstab brauchbares Festbettverfahren zu entwickeln, nach welchem sowohl Monoamino-methyl-cyclohexane als auch Diamino-methyl-cyclohexane in einem gewünschten Mengenverhältnis hergestellt werden können, bei welchem Verluste durch die unerwünschte Bildung von methyl- und aminosubstituierten Dicyclohexyl-aminen vermieden und bei welchem wieterhin eine möglichst hohe Lebensdauer des verwendeten Katalysators angestrebt wird.

Überraschenderweise wurde jetzt gefunden, daß die obengenannten Anforderungen durch den Einsatz eines Festbettkatalysators erfüllt werden, der als aktive Bestandteile Ruthenium, Verbindungen von Seltenerdmetallen, von Mangan und von Alkali- und/oder Erdalkalimetallen, bevorzugt von Erdalkalimetallen, enthält, die auf einen Al₂O₃-Träger aufgebracht werden.

Die Erfindung betrifft somit ein kontinuierliches Verfahren zur Herstellung eines Gemisches aus Amino-methyl-cyclohexanen und Diamino-methyl-cyclohexanen der Formeln 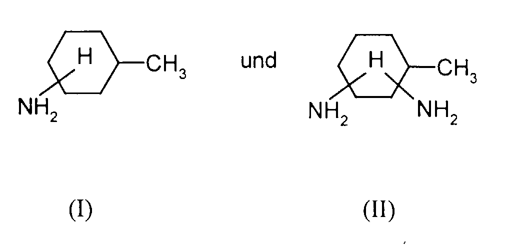 durch katalytische Hydrierung von Diamino-toluolen der Formel 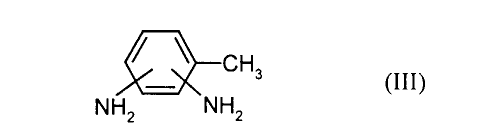 mit Wasserstoff bei Reaktionstemperaturen von 150 bis 260°C und einem H₂ -Druck von 20 bis 500 bar, das dadurch gekenzeichnet ist, daß als Katalysator Ruthenium mit Verbindungen von Seltenerdmetallen, von Mangan und von Alkalimetall-hydroxiden und/oder Erdalkalimetall-hydroxiden auf einem Al₂O₃-Träger eingesetzt wird.

Als Träger werden α- oder γ-Al₂O₃ bevorzugt γ-Al₂O₃ verwendet.

Der Träger wird mit einer oder mehreren Verbindungen von Seltenerdmetallen und des Mangans dotiert. Der Gehalt an Seltenerdmetallen und Mangan, gerechnet als Metall, beträgt zusammen 0,1 bis 8 Gew.-%, bevorzugt 0,3 bis 5 Gew.-%, bezogen auf das Gesamtgewicht des Katalysators. Das Gewichtsverhältnis von Seltenerdmetallen zu Mangan beträgt 5:1 bis 1:5, bevorzugt 2:1 bis 1:2. Als Seltenerdmetalle werden die Elemente der III. Nebengruppe des Periodensystems (Mendelejew), wie Scandium, Yttrium, Lanthan und die Lanthaniden verstanden. In bevorzugter Weise werden Yttrium, Lanthan, Cer, Praseodym, Neodym und Dysprosium, in besonders bevorzugter Weise Cer und Lanthan und in ganz besonders bevorzugter Weise Cer eingesetzt. Die Seltenerdmetalle kommen vielfach miteinander vergesellschaftet vor. Das ganz besonders bevorzugte Cer kann beispielsweise mit Lanthan, Praseodym, Neodym, Dysprosium oder mit Yttrium oder mit mehreren von ihnen vergesellschaftet sein. Eine solche Vergesellschaftung ist dem Fachmann im übrigen für alle genannten Seltenerdmetalle geläufig.

Das als weiterer Aktivbestandteil wirkende Edelmetall Ruthenium liegt in einer Gesamtmenge von 0,05 bis 5, bevorzugt 0,05 bis 4, besonders bevorzugt 0,1 bis 3 Gew.-%, bezogen auf das Gesamtgewicht des Katalysators, vor.

7ur Herstellung der erfindungsgemäß einzusetzenden Katalysatoren wird so vorgegangen, daß auf den Träger Aluminiumoxid in Form von Strangpreßlingen, Pillen oder Kugeln mit Durchmessern von etwa 2 bis 10 mm Verbindungen der Seltenerdmetalle und des Mangans aufgebracht werden. Der so dotierte Träger wird nach dem Trocknen auf 200 bis 450°C erhitzt und anschließend mit einer Lösung eines Rutheniumsalzes getränkt oder besprüht, wonach sich eine erneute Trocknungsphase anschließt.

Das Aufbringen von Verbindungen der Seltenerdmetalle und des Mangans auf den Katalysatorträger kann beispielsweise durch bloßes Tränken oder Aufsprühen mit wäßrigen Lösungen geeigneter Salze der Seltenerdmetalle und des Mangans erfolgen. Das Aufbringen von Verbindungen der Seltenerdmetalle und des Mangans kann jedoch auch durch gemeinsames Ausfällen eines Seltenerdmetall/Mangan-Hydroxidgemisches aus Seltenerdmetall- und Mangansalzen auf dem Träger mit Alkalilauge oder Ammoniak und gegebenenfalls anschließendes Auswaschen der löslichen Anteile mit Wasser erfolgen. Als Seltenerdmetall- und Mangansalze kommen insbesondere die Sulfate, Chloride, Acetate und/oder Nitrate der genannten Elemente in Betracht. Nach dem Aufbringen der Seltenerdmetall- und Mangansalze und gegebenenfalls nach der beschriebenen Ausfällung und der darauf erfolgenden Auswaschung wasserlöslicher Verbindungen wird der so behandelte Träger zunächst getrocknet, bevor er auf die genannte höhere Temperatur, etwa auf 200 bis 450°C, bevorzugt 250 bis 430°C, erhitzt wird. Dieses Erhitzen erfolgt in einer Zeit von 1 bis 120 Stunden. Während dieser Zeit kann die Temperatur im angegebenen Bereich von niederen auf höhere Werte erhöht werden.

Nach der beschriebenen Temperung wird der so dotierte Katalysatorträger mit einer Ruthenium enthaltenden Lösung getränkt. Hierbei kann so vorgegangen werden, daß das Ruthenium beispielsweise in Form wäßriger Lösungen des Chlorids, Nitrats, Acetats oder eines anderen geeigneten Salzes auf den Träger aufgetränkt oder aufgesprüht wird, gefolgt von einer Trocknung. Gegebenenfalls können die Rutheniumsalze auch in organischen Lösungsmitteln, wie Methanol, Acetonitril oder Dioxan, in Lösung gebracht und in dieser Weise aufgetränkt werden. Man kann jedoch auch vor der Trocknung den mit Rutheniumsalzen getränkten Träger mit einer wäßrigen Lösung der obengenannten basischen Verbindungen behandeln, wobei das Ruthenium als Oxid oder Hydroxid ausfällt. Auch nach dieser Variante der Aufbringung von Ruthenium schließt sich eine Trocknung an. Man kann jedoch auch den mit Verbindungen der Seltenerdmetalle und des Mangans beaufschlagten Katalysatorträger zunächst mit der Lösung einer der genannten basischen Verbindungen tränken, anschließend trocknen und auf den so vorbehandelten, basisch eingestellten Katalysatorträger Lösungen von Rutheniumsalzen auftragen, wobei im Moment der Tränkung auch die Ausfällung des Rutheniums in Form seines Oxids oder Hydroxids erfolgt.

Der einzusetzende Katalysator enthält weiterhin 1 bis 6 Gew.-%, bevorzugt 2 bis 5 Gew.-%, bezogen auf das Gesamtgewicht des Katalysators, eines oder mehrere Alkalimetallhydroxide oder 0,5 bis 10 Gew.-%, bevorzugt 1 bis 7 Gew.-%, ebenfalls bezogen auf das Gesamtgewicht des Katalysators, eines oder mehrere Erdalkalimetallhydroxide. Alkalimetallhydroxide sind Lithiumhydroxid, Natriumhydroxid, Kaliumhydroxid, Rubidiumhydroxid, Cäsiumhydroxid, bevorzugt Lithiumhydroxid, Natriumhydroxid, Kaliumhydroxid, besonders bevorzugt Natriumhydroxid oder Kaliumhydroxid. Erdalkalimetallhydroxide sind Berylliumhydroxid, Magnesiumhydroxid, Calciumhydroxid, Strontiumhydroxid, Bariumhydroxid oder Mischungen obiger Verbindungen, bevorzugt Calciumhydroxid, Strontiumhydroxid, Bariumhydroxid, besonders bevorzugt Strontiumhydroxid und/oder Bariumhydroxid.

Auch nach dem Auftragen der Alkalimetallhydroxide oder Erdalkalimetallhydroxide schließt sich eine Trocknung, im allgemeinen bei 100 bis 140°C bei vermindertem bis normalen Druck (1 bis 1000 mbar, bevorzugt 10 bis 500 mbar, beispielsweise im Wasserstrahlvakuum) an.

Das Auftränken des Rutheniums bzw. der Alkalimetallhydroxide oder Erdalkalimetallhydroxide erfolgt getrennt. Hierbei kann so vorgegangen werden, daß zunächst das Ruthenium in der oben beschriebenen Weise aufgetränkt wird, wobei nach einer Trocknung eine weitere Tränkung mit Alkalimetallhydroxiden oder Erdalkalimetallhydroxiden erfolgt. Bei dieser Behandlung wird das Ruthenium in Form seines Oxids oder Hydroxids ausgefällt. Die Alkalimetallhydroxide oder die Erdalkalimetallhydroxide können getrennt oder gemeinsam aufgetränkt werden. Man kann jedoch auch den Träger zunächst mit einer Alkalimetallhydroxidlösung oder einer Erdalkalimetallhydroxidlösung tränken, anschließend trocknen und auf den so vorbehandelten, basisch eingestellten Katalysatorträger Salze des Rutheniums auftragen, wobei im Moment der Tränkung auch die Ausfällung des Rutheniums in Form des Oxids oder Hydroxids erfolgt. Statt einer Tränkung mit den genannten Salzlösungen kann auch ein Besprühen erfolgen. Die hierzu erforderlichen Arbeitsgeräte und die Einstellung der gewünschten Beaufschlagung durch Wahl der Menge und Konzentration der Lösungen der genannten Stoffe sind dein Fachmann grundsätzlich bekannt.

Ein in der genannten Weise hergestellter Trägerkatalysator steht nach der letzten Trocknungsphase grundsätzlich für den erfindungsgemäßen Einsatz zur Verfügung. In bevorzugter Weise wird er jedoch vor seinem Einsatz, besonders bevorzugt nach Anordnung im Hydrierreaktor, durch Behandlung mit Wasserstoff bei einer Temperatur von 150 bis 400°C aktiviert.

Als Ausgangsstoffe für die Hydrierung nach dem erfindungsgemäßen Verfahren kommen beispielsweise in Betracht: 2,4-Diamino-toluol, 2,5-Diamino-toluol, 2,6-Diamino-toluol oder Mischungen aus diesen Verbindungen.

Mit Hilfe des Einsatzes der beschriebenen Katalysatoren entstehen in dem erfindungsgemäßen Verfahren Gemische aus Amino-methyl-cyclohexanen und Diamino-methyl-cyclohexanen, wobei überraschend in Abhängigkeit von der Hydriertemperatur das Mengenverhältnis der Amine so verändert werden kann, daß mit steigender Temperatur mehr Amino-methyl-cyclohexane gebildet werden und mit sinkender Temperatur der umgekehrte Effekt erreicht wird.

Das erfindungsgemäße Verfahren wird beispielsweise mit dem im Festbett angeordneten Katalysator in der Gas- bzw. Rieselphase durchgeführt. Es wird bei Wasserstoffüberschuß gearbeitet; die H₂-Menge beträgt das 10- bis 120-fache der Menge, bevorzugt die 10- bis 80-fache Menge, die für die Hydrierung eines Benzolkerns erforderlich ist.

Es wird bei 150 bis 260°C, bevorzugt bei 160 bis 250°C und bei einem Druck von mindestens 20 bar, bevorzugt mindestens 100 bar, besonders bevorzugt mindestens 200 bar gearbeitet. Die Obergrenze des zur Anwendung gelangenden Drucks ergibt sich sowohl aus technischen als auch aus wirtschaftlichen Überlegungen und liegt bei 500 bar, bevorzugt bei 200 bis 400 bar.

Als Katalysatorbelastung wird eine Menge von 0,05 bis 2 kg, bevorzugt 0,1 bis 1 kg, besonders bevorzugt 0,15 bis 0,6 kg Diamino-toluole pro Liter Katalysator pro Stunde eingestellt. Eine geringe Veränderung des erzielten Anteils an Amino-methyl-cyclohexanen durch veränderte Aktivität des Katalysators im Laufe besonders langer Reaktionsperioden kann durch ein geringes Nachstellen der Reaktionstemperatur oder der anderen Parameter ausgeglichen werden. Diese Verhältnisse können durch die Analytik des Reaktionsgemisches verfolgt werden.

Die Anordnung des erfindungsgemäß einzusetzenden Katalysators kann in verschiedenen, dem Fachmann für solche Zwecke grundsätzlich bekannten Apparaten erfolgen. In vorteilhafter Weise wird das erfindungsgemäße Verfahren in Röhrenreaktoren mit einer oder mehreren Röhren durchgeführt. Die Reaktionsrohre können Längen von beispielsweise 2 bis 20 m und innere Durchmesser von 20 bis 800 mm besitzen. Die Katalysatoren haben beispielsweise Abmessungen von 2 bis 10 mm und liegen beispielsweise als Strangpreßlinge, Pillen oder Kugeln vor.

Das erfindungsgemäße Verfahren kann mit oder ohne Lösungsmittel durchgeführt werden. Geeignete, unter den Reaktionsbedingungen inerte Lösungsmittel sind beispielsweise Methanol, Ethanol, Isopropanol.

Die erfindungsgemäß eingesetzten Katalysatoren zeigen sehr hohe Standzeiten; bisher sind 12.000 bis 15.000 Stunden beobachtet worden, nach denen die Versuche ohne erkennbares Nachlassen der Aktivität abgebrochen wurden.

Die nach der Hydrierung erhaltenen Reaktionsgemische enthalten praktisch keine methyl-substituierten Amino-di-N-cyclohexane, so daß besonders hohe Gehalte an Amino-methyl-cyclohexanen und Diamino-methyl-cyclohexanen erzielt werden können.

Die Hydriergemische können durch einfache Destillation aufgearbeitet werden. Für eine solche Aufarbeitung kann es vorteilhaft sein, die jeweiligen Diamino-toluole nicht vollständig umzusetzen, weil bei der Bildung der Monoamino-methyl-cyclohexane freiwerdendes Ammoniak sehr gut in den Diamino-toluolen löslich ist und so aus dem Reaktionsabgas entfernt werden kann. Bei der Destillation der Reaktionsprodukte wird das gelöste Ammoniak als erstes abdestilliert, kondensiert und damit für eine Weiterverwendung nutzbar; nicht vollständig umgesetzte Diamino-toluole können wieder in die Reaktion zurückgeführt werden. Auch der nicht verbrauchte Anteil des im Überschuß zugesetzten Wasserstoffs kann in die Reaktion zurückgeführt werden, wobei in vorteilhafter Weise der größte Teil dieses nicht umgesetzten Wasserstoffs in einem Hochdruckabscheider zurückgewonnen wird, so daß die Kompressionsarbeit für den Wasserstoff nicht noch einmal geleistet zu werden braucht.

Die erfindungsgemäß hergestellten Amino-methyl-cyclohexane und Diamino-methyl-cyclohexane werden nach erfolgreicher destillativer Trennung in einer Reinheit von mindestens 99,9 Gew.-% erhalten. In dieser Reinheit sind die genannten Verbindungen in der Regel für alle weiterverarbeitenden Prozesse einsetzbar.

Die Variabilität des erfindungsgemäßen Verfahrens zeigt sich in einer starken Zunahme des Anteils an Amino-methyl-cyclohexanen gegenüber den Diaminomethylcyclohexanen mit steigender Temperatur und sonst gleichen Bedingungen. So erhält man beispielsweise eine Steigerung des Anteils an Amino-methyl-cyclohexanen im Temperaturbereich von etwa 200 bis 230°C auf das 2- bis 10-fache des Anteils im Temperaturbereich von 170 bis 185°C.

### Beispiele

### Beispiel 1

2000 g eines handelsüblichen γ-Al₂O₃ mit einer spezifischen Oberfläche von 338 m²/g und einem Kugeldurchmesser von 2 bis 4 mm wurden mit einer Lösung getränkt, die aus 124 g Ce(NO₃)₃ o6 H₂O, 182,8 g Mn(NO₃)₂ o4 H₂O und 750 g Wasser hergestellt worden war. Das getränkte Al₂O₃ wurde bei 200 mbar 18 Stunden bei 120°C getrocknet und anschließend 8 Stunden lang bei 420°C getempert. 2000 g des so behandelten Katalysatorträgers wurden mit 700 g einer wäßrigen Ru(NO₃)₃-Lösung getränkt, die anteilig 20 g Ru enthielt. Der feuchte Katalysator wurde 18 Stunden bei 100°C unter einem Druck von 200 mbar getrocknet. 2000 g des mit Ru dotierten Katalysatorträgers wurden mit einer wäßrigen Ba(OH)₂-Aufschlämmung getränkt, die anteilig 30 g Ba enthielt. Der feuchte Katalysator wurde anschließend 20 Stunden bei 100°C unter einem Druck von 200 mbar getrocknet.

### Beispiel 2

2000 g eines handelsüblichen γ-Al₂O₃ mit einer spezifischen Oberfläche von 338 m²/g und einem Kugeldurchmesser von 2 bis 4 mm wurden mit einer Lösung imprägniert, die aus 125 g La(NO₃)₃ o6 H₂O, 178,5 g Mn(CH₃COO)₂ o4 H₂O und 400 g Wasser hergestellt worden war. Anschließend wurde das so imprägnierte Al₂O₃ 18 Stunden bei 100°C unter einem Druck von 200 mbar getrocknet und danach 5 Stunden bei 400°C getempert. 500 g des so behandelten Katalysatorträgers wurden mit 175 g einer wäßrigen Ru(NO₃)₃-Lösung getränkt, die anteilig 5 g Ru enthielt. Der feuchte Katalysator wurde 18 Stunden bei 100°C unter einem Druck von 200 mbar getrocknet. 500 g des mit Ru dotierten Katalysatorträgers wurden mit einer wäßrigen Sr(OH)₂-Aufschlämmung getränkt, die anteilig 5 g Sr enthielt. Der feuchte Katalysator wurde anschließend 20 Stunden bei 100°C unter einem Druck von 200 mbar getrocknet.

### Beispiel 3

Ein senkrecht stehendes, wärmeisoliertes Hochdruckrohr aus nichtrostendem Stahl von 30 mm Innendurchmesser und 1 m Länge, das vorher mit Stickstoff sauerstofffrei gespült worden war, wurde mit 400 ml eines kugelförmigen Ru/Ce/Mn/Ba-Al₂O₃-Katalysators, der nach Beispiel 1 hergestellt worden war und 1 Gew.-% Ru, 2 Gew.-% Ce, 2 Gew.-% Mn und 1,5 Gew.-% Ba enthielt, gefüllt. Zur Aktivierung des Katalysators wurden die Katalysatorkugeln zunächst 6 Stunden im Stickstoffstrom nachgetrocknet (Temperatur: max. 200°C, Menge 1,5 Nm³ N₂/h). Die eigentliche Aktivierung erfolgte unter einem Stickstoffdruck von 200 bar bei einer Temperatur zwischen 150 und 350°C, wobei dem Stickstoff allmählich Wasserstoff zugemischt wurde. Innerhalb von 12 Stunden wurde der Stickstoffanteil mehr und mehr vermindert, bis schließlich nur Wasserstoff den Reaktor durchströmte.

Nach der Aktivierung des Hydrierkatalysators wurde der Wasserstoffdruck im Reaktorsystem auf 300 bar erhöht. Anschließend wurden stündlich 100 g 2,4-Diamino-toluol gemeinsam mit 1000 Nl Wasserstoff unter einem Druck von 300 bar von oben nach unten absteigend durch das Hochdruckrohr gepumpt, wobei das 2,4-Diamino-toluol vor Eintritt in den Reaktor in einem vorgeschalteten, elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur von <60°C abgekühlt und in einem Gasabscheider von überschüssigem Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angaben in Flächen-%; der Rest zu 100 % sind Nebenprodukte):

| Laufzeit (h) | Temperatur (°C) | 2- und 4-Amino-methyl-cyclohexan (F-%) | 2,4-Diamino-methyl-cyclohexan (F-%) | 2,4-Diamino-toluol (F-%) |
|---|---|---|---|---|
| 116 | 175 | 5,2 | 34,9 | 57,9 |
| 144 | 180 | 13,9 | 40,3 | 44,3 |
| 198 | 205 | 24,8 | 57,0 | 16,4 |
| 224 | 215 | 33,0 | 62,8 | 2,37 |
| 368 | 220 | 43,3 | 56,5 | 0,16 |

### Beispiel 4

Ein senkrecht stehendes, wärmeisoliertes Hochdruckrohr aus nichtrostendem Stahl von 30 mm Innendurchmesser und 1 m Länge, das vorher mit Stickstoff sauerstofffrei gespült worden war, wurde mit 400 ml eines kugelförmigen Ru/La/Mn/Sr-Al₂O₃-Katalysators, der nach Beispiel 2 hergestellt worden war und 1 Gew.-% Ru, 2 Gew.-% La, 2 Gew.-% Mn und 1 Gew.-% Sr enthielt, gefüllt. Zur Aktivierung des Katalysators wurden die Katalysatorkugeln zunächst 8 Stunden im Stickstoffstrom nachgetrocknet (Temperatur: max. 200°C, Menge 1,5 Nm³ N₂/h. Die eigentliche Aktivierung erfolgte unter einem Stickstoffdruck von 200 bar bei einer Temperatur zwischen 150 und 350°C, wobei dem Stickstoff allmählich Wasserstoff zugemischt wurde. Innerhalb von 12 Stunden wurde der Stickstoffanteil mehr und mehr vermindert, bis schließlich nur Wasserstoff den Reaktor durchströmte.

Nach der Aktivierung des Hydrierkatalysators wurde der Wasserstoffdruck im Reaktorsystem auf 300 bar erhöht. Anschließend wurden stündlich 100 g 2,6-Diamino-toluol gemeinsam mit 1000 Nl Wasserstoff unter einem Druck von 300 bar von oben nach unten absteigend durch das Hochdruckrohr gepumpt, wobei das 2,6-Diamino-toluol vor Eintritt in den Reaktor in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur von <60°C abgekühlt und in einem Gasabscheider von überschüssigem Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angabe in Flächen-%; der Rest zu 100 % sind Nebenprodukte):

| Laufzeit (h) | Temperatur (°C) | 2-Amino-methyl-cyclohexan (F-%) | 2,6-Diamino-methyl-cyclohexan (F-%) | 2,6-Diamino-toluol (F-%) |
|---|---|---|---|---|
| 92 | 175 | 8,6 | 34,3 | 55,6 |
| 224 | 180 | 15,2 | 41,1 | 41,9 |
| 418 | 205 | 27,2 | 55,6 | 15,2 |
| 466 | 215 | 36,2 | 58,6 | 3,1 |
| 512 | 230 | 48,1 | 50,3 | 0,2 |

### Beispiel 5

Ein senkrecht stehendes, wärmeisoliertes Hochdruckrohr aus nichtrostendem Stahl von 30 mm Innendurchmesser und 1 m Länge, das vorher mit Stickstoff sauerstofffrei gespült worden war, wurde mit 400 ml eines kugelförmigen Ru/Ce/Mn/Ba-Al₂O₃-Katalysators, der nach Beispiel 1 hergestellt worden war und 1 Gew.-% Ru, 2 Gew.-% Ce, 2 Gew.-% Mn und 1,5 Gew.-% Ba enthielt, gefüllt. Zur Aktivierung des Katalysators wurden die Katalysatorkugeln zunächst 6 Stunden im Stickstoffstrom nachgetrocknet (Temperatur: max. 200°C, Menge 1,5 Nm³ N₂/h). Die eigentliche Aktivierung erfolgte unter einem Stickstoffdruck von 200 bar bei einer Temperatur zwischen 150 und 350°C, wobei dem Stickstoff allmählich Wasserstoff zugemischt wurde. Innerhalb von 12 Stunden wurde der Stickstoffanteil mehr und mehr vermindert, bis schließlich nur Wasserstoff den Reaktor durchströmte.

Nach der Aktivierung des Hydrierkatalysators wurde der Wasserstoffdruck im Reaktorsystem auf 300 bar erhöht. Anschließend wurden stündlich 80 g eines Gemisches aus 80 Gew.-% 2,4-Diamino-toluol und 20 Gew.-% 2,6-Diamino-toluol gemeinsam mit 1000 Nl Wasserstoff unter einem Druck von 300 bar von oben nach unten absteigend durch das Hochdruckrohr gepumpt, wobei das Diamino-toluol-Gemisch vor Eintritt in den Reaktor in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur von <60°C abgekühlt und in einem Gasabscheider von überschüssigem Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angabe in Flächen-%; der Rest zu 100 % sind Nebenprodukte):

| Laufzeit (h) | Temperatur (°C) | 2- und 4-Amino-methyl-cyclohexan (F-%) | 2,4- und 2,6-Diamino-methyl-cyclohexan (F-%) | 2,4- und 2,6-Diamino-toluol (F-%) |
|---|---|---|---|---|
| 566 | 210 | 23,9 | 63,5 | 12,9 |
| 1938 | 155 | 3,1 | 19,4 | 76,1 |
| 2008 | 160 | 5,2 | 27,8 | 65,3 |
| 2056 | 165 | 8,9 | 39,1 | 49,6 |
| 2154 | 185 | 10,1 | 44,9 | 44,4 |
| 2176 | 195 | 16,1 | 53,1 | 29,8 |
| 2224 | 200 | 16,4 | 61,0 | 22,1 |
| 2272 | 210 | 22,8 | 63,7 | 13,2 |
| 2424 | 220 | 33,0 | 65,1 | 1,1 |

## Patentansprüche

1. Kontinuierliches Verfahren zur Herstellung eines Gemisches aus Amino-methyl-cyclohexanen und Diamino-methyl-cyclohexanen der Formeln 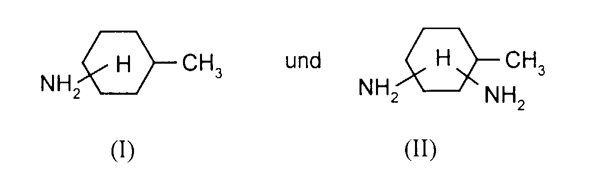 durch katalytische Hydrierung von Diamino-toluolen der Formel 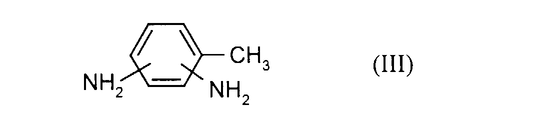 mit Wasserstoff bei Reaktionstemperaturen von 150 bis 260°C und einem H₂-Druck von 20 bis 500 bar, dadurch gekennzeichnet, daß als Katalysatoren Ruthenium, Mangan, Verbindungen von Seltenerdmetallen und Alkalimetallhydroxide und/oder Erdalkalimetall-hydroxide enthaltende α-Al₂O₃- oder γ-Al₂O₃-Träger, die aus Strangpreßlingen, Pillen oder Kugeln mit Abmessungen von etwa 2-10 mm bestehen, eingesetzt werden.

2. Verfahren nach Anspruch 1, dadurch gekennzeichnet, daß der Träger γ-Al₂O₃ ist.

3. Verfahren nach Anspruch 1, dadurch gekennzeichnet, daß auf einen γ-Al₂O₃-Träger eine oder mehrere Verbindungen von Seltenerdmetallen und des Mangans aufgetragen werden, wobei der Gehalt von Seltenerdmetallen und Mangan, gerechnet als Metall, zusammen 0,1 bis 8 Gew.-%, bevorzugt 0,3 bis 5 Gew.-%, bezogen auf das Gesamtgewicht des Katalysators, beträgt, wobei das Gewichtsverhältnis von seltenen Erdmetallen zu Mangan 5:1 bis 1:5, bevorzugt 2:1 bis 1:2 beträgt und wobei als Seltenerdmetalle die Elemente der III. Nebengruppe des Periodensystems (Mendelejew), wie Scandium, Yttrium, Lanthan und die Lanthaniden, in bevorWeise Yttrium, Lanthan, Cer, Praseodym, Neodym und Dysprosium, in besonders bevorzugter Weise Cer und Lanthan und in ganz besonders bevorzugter Weise Cer aufgetragen werden/wird.

4. Verfahren nach Anspruch 1, wonach auf einen γ-Al₂O₃-Träger Ruthenium in einer Gesamtmenge von 0,05 bis 5, bevorzugt 0,05 bis 4, besonders bevorzugt 0,1 bis 3 Gew.-%, bezogen auf das Gesamtgewicht des Katalysators, aufgetragen wird.

5. Verfahren nach Anspruch 1, wonach auf einen γ-Al₂O₃-Träger ein oder mehrere Alkalimetallhydroxide in einer Gesamtmenge von 1 bis 6 Gew.-%, bevorzugt 2 bis 5 Gew.-%, bezogen auf das Gesamtgewicht, oder ein oder mehrere Erdalkalimetallhydroxide in einer Gesamtmenge von 0,5 bis 10 Gew.-%, bevorzugt 1 bis 7 Gew.-%, ebenfalls bezogen auf das Gesamtgewicht, aufgetragen werden, wobei als Alkalimetallhydroxid(e) Lithiumhydroxid, Natriumhydroxid, Kaliumhydroxid, Rubidiumhydroxid, Cäsiumhydroxid, bevorzugt Lithiumhydroxid, Natriumhydroxid, Kaliumhydroxid, besonders bevorzugt Natriumhydroxid oder Kaliumhydroxid eingesetzt und als Erdalkalimetallhydroxid(e) Berylliumhydroxid, Magnesiumhydroxid, Calciumhydroxid, Strontiumhydroxid, Bariumhydroxid, bevorzugt Calciumhydroxid, Strontiumhydroxid, Bariumhydroxid, besonders bevorzugt Strontiumhydroxid und/oder Bariumhydroxid eingesetzt werden/wird.

6. Verfahren nach Anspruch 1, dadurch gekennzeichnet, daß bei einem H₂-Druck von 100 bis 400 bar, bevorzugt 200 bis 400 bar gearbeitet wird.

7. Verfahren nach Anspruch 1, dadurch gekennzeichnet, daß bei einer Temperatur von 160 bis 250°C gearbeitet wird.

8. Verfahren nach Anspruch 1, dadurch gekennzeichnet, daß kontinuierlich in der Gas- oder Rieselphase an fest angeordneten Katalysatoren gearbeitet wird und eine Katalysatorbelastung von 0,05 bis 2 kg, bevorzugt 0,1 bis 1 kg, besonders bevorzugt 0,15 bis 0,6 kg Diamino-toluole pro Liter Katalysator pro Stunde eingestellt wird.

9. Verfahren nach Anspruch 1, dadurch gekennzeichnet, daß der Katalysator vor seinem Einsatz, bevorzugt nach Anordnung im Hydrierreaktor durch Behandlung mit Wasserstoff bei einer Temperatur von 100 bis 400°C, bevorzugt 150 bis 350°C, aktiviert wird.

## Claims

1. Continuous p:cocess for preparing a mixture of amino-methyl-cyclohexanes and diamino-methylcyclohexanes of the formulae 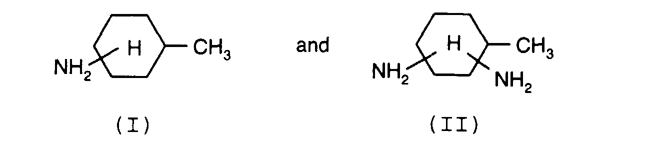 by catalytic hydrogenation of diamino-toluenes of the formula 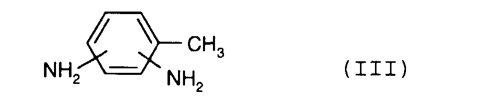 with hydrogen at reaction temperatures of from 150 to 260°C and an H₂ pressure of from 20 to 500 bar, characterized in that the catalysts used are α-Al₂O₃ or γ-Al₂O₃ supports which comprise ruthenium, manganese, or compounds of rare earth metals, and alkali metal hydroxides and/or alkaline earth metal hydroxides and which consist of extrudates, pellets or spheres having dimensions of about 2-10 mm.

2. Process according to Claim 1, characterized in that the support is γ-Al₂O₃.

3. Process according to Claim 1, characterized in that one or more compounds of rare earth metals and of manganese are applied to a γ-Al₂O₃ support and the content of rare earth metals and manganese, calculated as metal, is together from 0.1 to 8% by weight, preferably from 0.3 to 5% by weight, based on the total weight of the catalyst, the weight ratio of rare earth metals to manganese is from 5:1 to 1:5, preferably from 2:1 to 1:2 and the rare earth metals applied are the elements of transition group III of the Periodic Table (Mendeleev), for example scandium, yttrium, lanthanum and the lanthanides, preferably yttrium, lanthanum, cerium, praseodymium, neodymium and dysprosium, particularly preferably cerium and lanthanum and very particularly preferably cerium.

4. Process according to Claim 1, wherein a γ-Al₂O₃ support has ruthenium applied to it in a total amount of from 0.05 to 5, preferably from 0.05 to 4, particularly preferably from 0.1 to 3,% by weight, based on the total weight of the catalyst.

5. Process according to Claim 1, wherein a γ-Al₂O₃ support has one or more alkali metal hydroxides applied to it in a total amount of from 1 to 6% by weight, preferably from 2 to 5% by weight, based on the total weight, or one or more alkaline earth metal hydroxides in a total amount of from 0.5 to 10% by weight, preferably from 1 to 7% by weight, likewise based on the total weight, where the alkali metal hydroxide(s) is/are lithium hydroxide, sodium hydroxide, potassium hydroxide, rubidium hydroxide, caesium hydroxide, preferably lithium hydroxide, sodium hydroxide, potassium hydroxide, particularly preferably sodium hydroxide or potassium hydroxide and the alkaline earth metal hydroxide(s) is/are beryllium hydroxide, magnesium hydroxide, calcium hydroxide, strontium hydroxide, barium hydroxide, preferably calcium hydroxide, strontium hydroxide, barium hydroxide, particularly preferably strontium hydroxide and/or barium hydroxide.

6. Process according to Claim 1, characterized in that it is carried out at an H₂ pressure of from 100 to 400 bar, preferably from 200 to 400 bar.

7. Process according to Claim 1, characterized in that it is carried out at a temperature of from 160 to 250°C.

8. Process according to Claim 1, characterized in that it is carried out continuously in the gas phase or trickle phase over fixed-bed catalysts at a weight hourly space velocity over the catalyst of from 0.05 to 2 kg, preferably from 0.1 to 1 kg, particularly preferably from 0.15 to 0.6 kg, of diamino-toluenes per litre of catalyst per hour.

9. Process according to Claim 1, characterized in that the catalyst is activated by treatment with hydrogen at a temperature of from 100 to 400°C, preferably from 150 to 350°C, before use, preferably after installation in the hydrogenation reactor.

## Revendications

1. Procédé en continu pour la préparation d'un mélange d'aminométhylcyclohexanes et de diaminométhylcyclohexanes répondant aux formules 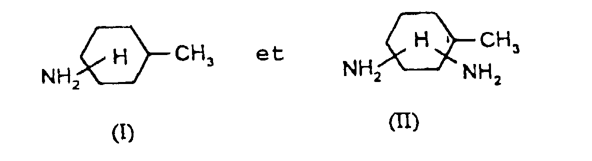 par hydrogénation catalytique de diamino-toluènes répondant à la formule 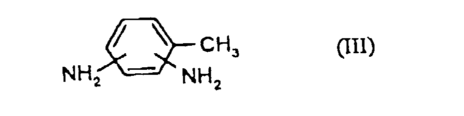 avec de l'hydrogène à des températures réactionnelles de 150 à 260°C et sous une pression de H₂ de 20 à 500 bar, caractérisé en ce qu'on met en oeuvre, à titre de catalyseurs, des supports de α-Al₂O₃ ou de γ-Al₂O₃ contenant du ruthénium, du manganèse, des composés de métaux des terres rares et d'hydroxydes de métaux alcalins et/ou d'hydroxydes de métaux alcalino-terreux, qui sont constitués par des extrudats, des pastilles ou des sphères possédant des dimensions d'environ 2 à 10 mm.

2. Procédé selon la revendication 1, caractérisé en ce que le support est le γ-Al₂O₃.

3. Procédé selon la revendication 1, caractérisé en ce qu'on applique, sur un support de γ-Al₂O₃, un ou plusieurs composés de métaux des terres rares et du manganèse, la teneur des métaux des terres rares et du manganèse, calculée comme métal, s'élève de manière conjointe de 0,1 à 8% en poids, de préférence de 0,3 à 5% en poids rapportés au poids total du catalyseur, dans lequel le rapport pondéral des métaux des terres rares au manganèse s'élève de 5:1 à 1:5, de préférence de 2:1 à 1:2, et dans lequel on applique, à titre de métaux des terres rares, les éléments du IIIème sous-groupe du système périodique (Mendelejew) tels que le scandium, l'yttrium, le lanthane et les lanthanides, de préférence l'yttrium, le lanthane, le cérium, le praséodyme, le néodyme et le dysprosium, de manière particulièrement préférée le cérium et le lanthane, de manière tout particulièrement préférée le cérium.

4. Procédé selon la revendication 1, selon lequel on applique, sur un support de γ-Al₂O₃, du ruthénium en une quantité totale de 0,05 à 5, de préférence de 0,05 à 4, de manière particulièrement préférée de 0,1 à 3% en poids rapportés au poids total du catalyseur.

5. Procédé selon la revendication 1, selon lequel on applique, sur un support de γ-Al₂O₃, un ou plusieurs hydroxydes de métaux alcalins en une quantité totale de 1 à 6% en poids, de préférence de 2 à 5% en poids rapportés au poids total ou bien un ou plusieurs hydroxydes de métaux alcalino-terreux en une quantité totale de 0,5 à 10% en poids, de préférence de 1 à 7% en poids également rapportés au poids total, dans lequel on met en oeuvre, à titre d'hydroxyde(s) de métaux alcalins, l'hydroxyde de lithium, l'hydroxyde de sodium, l'hydroxyde de potassium, l'hydroxyde de rubidium, l'hydroxyde de césium, de préférence l'hydroxyde de lithium, l'hydroxyde de sodium, l'hydroxyde de potassium, de manière particulièrement préférée l'hydroxyde de sodium ou l'hydroxyde de potassium et, à titre d'hydroxyde(s) de métaux alcalino-terreux, l'hydroxyde de béryllium, l'hydroxyde de magnésium, l'hydroxyde de calcium, l'hydroxyde de strontium, l'hydroxyde de baryum, de préférence l'hydroxyde de calcium, l'hydroxyde de strontium, l'hydroxyde de baryum, de manière particulièrement préférée l'hydroxyde de strontium et/ou l'hydroxyde de baryum.

6. Procédé selon la revendication 1, caractérisé en ce qu'on travaille sous une pression de H₂ de 100 à 400 bar, de préférence de 200 à 400 bar.

7. Procédé selon la revendication 1, caractérisé en ce qu'on travaille à une température de 160 à 250°C.

8. Procédé selon la revendication 1, caractérisé en ce qu'on travaille en continu en phase gazeuse ou en phase de ruissellement sur des catalyseurs en lit fixe et on règle une charge du catalyseur de 0,05 à 2 kg, de préférence de 0,1 à 1 kg, de manière particulièrement préférée de 0,15 à 0,6 kg de diaminotoluènes par litre du catalyseur par heure.

9. Procédé selon la revendication 1, caractérisé en ce qu'on active le catalyseur avant sa mise en oeuvre, de préférence après sa mise en place dans le réacteur d'hydrogénation, par traitement avec de l'hydrogène à une température de 100 à 400°C, de préférence de 150 à 350°C.
